(19) Europäisches Patentamt
European Patent Office
Office européen des brevets

(11) Veröffentlichungsnummer: **0 263 481 B1**

(12) # EUROPÄISCHE PATENTSCHRIFT

(45) Veröffentlichungstag der Patentschrift: **02.01.92**

(51) Int. Cl.⁵: **C07C 263/00**

(21) Anmeldenummer: **87114555.3**

(22) Anmeldetag: **06.10.87**

(54) **Verfahren zur Herstellung von Isocyanaten.**

(30) Priorität: **08.10.86 DE 3634248**

(43) Veröffentlichungstag der Anmeldung:
**13.04.88 Patentblatt 88/15**

(45) Bekanntmachung des Hinweises auf die Patenterteilung:
**02.01.92 Patentblatt 92/01**

(84) Benannte Vertragsstaaten:
**BE DE ES FR GB IT NL**

(56) Entgegenhaltungen:
**DE-B- 1 668 076**
**DE-B-16 680 69**

(73) Patentinhaber: **BAYER AG**

**W-5090 Leverkusen 1 Bayerwerk(DE)**

(72) Erfinder: **Mormann, Werner, Prof. Dr.**
**Zum Wolfsloch 30**
**W-5910 Kreuztal(DE)**
Erfinder: **Hissmann, Edith**
**Augustdorfer Strasse 40**
**W-4815 Schloss Holte-Stukenbrock(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein neues Verfahren zur Herstellung von Ester- und/oder Amidgruppen aufweisenden Isocyanaten unter Verwendung von Isocyanatoalkylcarbonsäurechloriden und organischen Hydroxy- und/oder Aminoverbindungen in silylierter Form als Ausgangsmaterialien.

Die Umsetzung von Isocyanatocarbonsäurechloriden mit Alkoholen oder Aminen ist bekannt (Iwakura et. al. J. Org. Chem. 31 (1966), 142). Die Umsetzung ist jedoch wenig selektiv; so resultieren beispielsweise bei der Umsetzung eines Isocyanatocarbonsäurechlorids mit Ethanol im Molverhältnis 1:1 Isocyanatocarbonsäureester im Gemisch mit dem entsprechenden Urethan und unverändertem Isocyanatocarbonsäurechlorid:

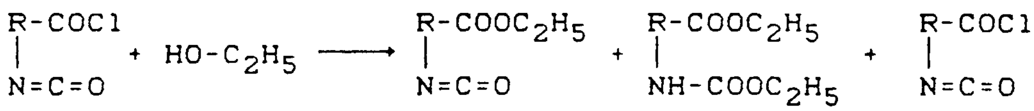

Diese mangelnde Selektivität hat einer technischen Verwendung bisher im Wege gestanden.

Wie jetzt überraschend gefunden wurde, gelingt es Isocyanatoalkylcarbonsäurechloride dann selektiv in die entsprechenden Isocyanatoalkylcarbonsäureester bzw. -amide überzuführen, wenn als Reaktionspartner nicht die freien Hydroxylgruppen bzw. Aminogruppen aufweisenden Verbindungen, sondern deren silylierte Derivate verwendet werden.

Gegenstand der Erfindung ist somit ein Verfahren zur Herstellung von Ester- und/oder Amidgruppen aufweisenden Isocyanaten, dadurch gekennzeichnet, daß man

a) Isocyanatoalkylcarbonsäurechloride

mit

b) organischen Verbindungen, die mindestens eine silylierte alkoholische und/oder phenolische Hydroxylgruppe und/oder mindestens eine silylierte Aminogruppen aufweisen und ansonsten unter den jeweiligen Reaktionsbedingungen gegenüber Isocyanat- und Chlorcarbonylgruppen inert sind,

bei -20 °C bis +150 °C zur Reaktion bringt.

Als Komponente a) können beim erfindungsgemäßen Verfahren beliebige Isocyanatoalkylcarbonsäurechloride eingesetzt werden, die, von der Chlorcarbonylgruppe abgesehen, gegenüber silylierten Hydroxyl- oder Aminogruppen inert sind. Gut geeignet sind beispielsweise Verbindungen der allgemeinen Formel

OCN-R-COCl

für welche

R für einen aliphatischen Kohlenwasserstoffrest mit 2 bis 5 Kohlenstoffatomen steht.

Beispiel für derartige besonders bevorzugte Isocyanatocarbonsäurechloride sind 3-Isocyanatopropionsäurechlorid, 4-Isocyanatobuttersäurechlorid oder 6-Isocyanatocapronsäurechlorid.

Neben diesen besonders bevorzugt beim erfindungsgemäßen Verfahren einzusetzenden Isocyanatocarbonsäurechloriden können auch solche der zuletzt genannten allgemeinen Formel verwendet werden, für welche R für einen aliphatischen Kohlenwasserstoffrest mit mehr als 6 Kohlenstoffatomen steht.

Beispiel für derartige Isocyanatoalkylcarbonsäurechloride ist 12-Isocyanatododecansäurechlorid, Ferner geeignet sind solche Isocyanatoalkylcarbonsäurechloride die mehr als eine Isocyanatgruppe und/oder mehr als eine Carbonsäurechloridgruppe aufweisen. Hierzu gehören beispielsweise 2,6-Diisocyanato-capronsäurechlorid oder 2-Isocyanatoglutarsäure-dichlorid.Die Verwendung derartiger mehr als eine Isocyanat- und/oder Chorcarbonylgruppe aufweisender Verbindungen ist allerdings weniger bevorzugt.

Als Komponente b) können beim erfindungsgemäßen Verfahren beliebige organische Verbindungen eingesetzt werden, die mindestens eine alkoholische oder phenolische Hydroxylgruppe in silylierter Form und/oder mindestens eine primäre oder sekundäre Aminogruppe in silylierter Form aufweisen, und die, von diesen Gruppierungen abgesehen, gegenüber Isocyanat- und Chlorcarbonylgruppen inert sind. Besonders gut geeignet sind beispielsweise Verbindungen der allgemeinen Formel

$[R'_3Si-X]_mR''$

in welcher

R' für einen aliphatischen Kohlenwasserstoffrest mit 1 bis 4 Kohlenstoffatomen, vorzugsweise eine Methylgruppe steht,

R″ für einen jeweils m-wertigen aliphatischen Kohlenwasserstoffrest mit 1 bis 18, vorzugsweise 1 bis 6 Kohlenstoffatomen, cycloaliphatischen Kohlenwasserstoffrest mit 4 bis 15, vorzugsweise mit 6 bis 13 Kohlenstoffatomen oder aromatischen Kohlenwasserstoffrest mit 6 bis 13 Kohlenstoffatomen steht,

X für Sauerstoff oder eine Gruppierung der Formel - NR‴- steht, wobei R‴ für Wasserstoff oder einen Alkylrest, insbesondere einen Methylrest und besonders bevorzugt für Wasserstoff steht, und

m eine ganze Zahl von 1 bis 4, insbesondere von 2 bis 4 bedeutet.

Als Komponente b) kommen somit beispielsweise an den alkoholischen Sauerstoffatomen Trialkylsilyl-substituierte primäre, sekundäre oder tertiäre Alkohole wie Methanol, Ethanol, n-Butanol, Isobutanol, tert.-Butanol, 1,4-Dihydroxybutan, 1,6-Dihydroxyhexan, Neopentylglykol, Trimethylolpropan oder Pentaerythrit, an den phenolischen Hydroxylgruppen Trialkylsilyl-substituierte Phenole wie Phenol, Kresol, Bisphenol-A, 4,4′-Dihydroxy-diphenyl, Hydrochinon, Resorcin, 4-Hydroxy-benzoesäurehydrochinon-monoester, 4,4′-Dihydroxy-diphenylsulfon, 1,5-, 2,6-, 2,7-Dihydroxynaphthalin, 2,2-Bis-(35-dichlor-4- hydroxyphenyl)-propan, 1,1-Bis-(4-hydroxyphenyl)-cyclohexan, 2,2′-Dihydroxy-1,1′-binaphthyl, 1,3,5-Trihydroxybenzol oder Isocyanatophenole wie p-Hydroxyphenylisocyanat, an den Aminogruppen Trialkylsilyl-substituierte primäre oder sekundäre Amine wie n-Butylamin, Anilin, 1,2-Diaminoethan, 1,4-Diaminobutan, 1,6-Diaminohexan, 4,4′-Diaminodiphenylmethan, 2,4-Diaminotoluol, 2,6- Diaminotoluol, 1,4-Diaminobenzol, 1,4-Diaminocyclohexan, Bis-(aminomethyl)-hexahydro-4,7-methano-indan, 4,4′-Diaminodiphenylethan, 4,4′-Diamino-diphenylsulfon,1,4-, 1,5-, 2,6-, 2,7-Diaminonaphthalin, 2,2′,5,5′-Tetrachlorbenzidin, 3,3′-Dimethyl-benzidin, 2,2-Bis-(4-aminophenyl)-propan, 1,1-Bis-(4-aminophenyl)-cyclohexan oder N- Methylanilin in Betracht. Ferner können als Komponente b) an den Stickstoff- und Sauerstoffatomen silylierte Aminoalkohole wie Aminoethanol oder 2-Aminobutanol, heterocyclische Verbindungen mit sekundären Aminogruppen in silylierter Form, wie beispielsweise silyliertes Piperazin oder auch substituierte Alkohole oder Amine mit silylierten Hydroxyl- oder Aminogruppen wie beispielsweise silyliertes Chlorethanol oder silylierte Aminocarbonsäuren eingesetzt werden.

Die Silylierung der in silylierter Form als Komponete b) einzusetzenden Ausgangsmaterialien erfolgt in an sich bekannter Weise durch Umsetzung der entsprechenden Hydroxyl- und/oder Aminogruppen aufweisenden Verbindungen mit Chlorsilanen oder Disilazanen der allgemeinen Formeln

$R'_3SiCl$

bzw.

$R'_3Si\text{-}NH\text{-}SiR'_3$.

In den beiden letztgenannten allgemeinen Formeln hat R′ die obengenannte Bedeutung. Jedoch muß darauf verwiesen werden, daß die Natur des Restes R′ für die Durchführbarkeit des erfindungsgemäßen Verfahrens ohne größere Bedeutung ist, da auch beispielsweise die entsprechenden Triarylsilyl-Derivate als Komponente b), eingesetzt werden könnten, was allerdings weniger bevorzugt ist.

Die Silylierung der Hydroxyl- und/oder Aminogruppen aufweisenden Ausgangsmaterialien erfolgt nach an sich bekannten Methoden und wird beispielsweise von M. Lalonde und C.H. Chan in Synthesis (1985), Seiten 817-845 beschrieben.

Die beim erfindungsgemäßen Verfahren ablaufende Reaktion kann mit nachstehender Gleichung beschrieben werden:

$$m\ OCN\text{-}R\text{-}COCl\ +\ [R'_3Si\text{-}X]_m R''$$
$$\rightarrow [OCN\text{-}R\text{-}CO\text{-}X]_m R''\ +\ m\ R'_3SiCl$$

Bei der Durchführung des erfindungsgemäßen Verfahrens wird die Menge der Reaktionspartner a) und b) im allgemeinen so gewählt, daß auf jedes Mol an Chlorcarbonylgruppen der Komponente a) mindestens 0,8 Mol an silylierten Hydroxyl- und/oder Aminogruppen entfallen. Vorzugsweise werden die Mengen der Reaktionspartner so gewählt, daß auf jedes Mol an Chlorcarbonyl-Gruppen der Komponente a) 0,8 bis 1,2 Mol an silylierten Amino-und/oder Hydroxylgruppen entfallen. Vorzugsweise wird unter Einhaltung von äqimolaren Mengen (Mol-Verhältnis der Reaktivgruppen = 1:1) gearbeitet. Die Verwendung eines über den Bereich von 0,8 bis 1,2 Mol hinausgehenden Überschusses einer der beiden Komponenten wäre zwar möglich, würde jedoch lediglich zu Ausbeuteverlusten führen. Lediglich im Sonderfall einer gegebenenfalls erwünschten selektiven Umsetzung, beispielsweise von Aminoalkoholen mit silylierten Amino-und Hydroxylgruppen zwecks Herstellung von silylierte Hydroxylgruppen aufweisenden Isocyanatocarbonsäureamiden bleiben die silylierten Hydroxylgruppen bei der Berechnung der Mengenverhältnisse der Reaktionspartner entsprechend den gemachten Ausführungen unberücksichtigt.

Als Nebenprodukt entsteht bei der erfindungsgemäßen Umsetzung das entsprechende Chlorsilan, das leicht durch Destillation abgetrennt und für eine erneute Silylierung eingesetzt werden kann.

Die erfindungsgemäße Umsetzung erfolgt im Falle der silylierten Alkohole und Phenole im allgemeinen innerhalb des Temperaturbereichs von 50 bis 150 °C, wobei man das Ende der Reaktion am Verschwinden der Säurechloridcarbonylbande bei 1.800 $cm^{-1}$ im Infrarotspektrum leicht erkennen kann.

Die entsprechende Umsetzung der silylierten Amine findet im allgemeinen innerhalb des Temperaturbereichs von -20 °C bis +50 °C, vorzugsweise von -10 °C bis +20 °C, statt.

Die Reaktion kann in Ab- oder Anwesenheit eines geeigneten Lösungsmittels durchgeführt werden. Geeignete Lösungsmittel sind beispielsweise Diethylether, Toluol, Xylol, Trichlorethylen, Essigsäureethylester, Essigsäurebutylester oder beliebige Gemische derartiger Lösungsmittel.

Die nach dem erfindungsgemäßen Verfahren erhältlichen Ester- oder Amidgruppen aufweisenden Monoisocyanate (m = 1) stellen interessante Zwischenprodukte für organische Synthesen, insbesondere von Schädlingsbekämpfungsmitteln dar. Die nach dem erfindungsgemäßen Verfahren erhältlichen Polyisocyanate (m = 2-4) stellen wertvolle Ausgangsmaterialien für die Herstellung von Polyurethankunststoffen dar. Die erfindungsgemäßen Verfahrensprodukte eignen sich ausgezeichnet zur Herstellung von Ein- oder Zweikomponenten-Polyurethanlacken. Die Funktionalität der nach dem erfindungsgemäßen Verfahren erhältlichen Polyisocyanate kann nicht nur durch entsprechende Wahl der Ausgangsmaterialien a) und b), sondern auch gegebenenfalls durch Verwendung von Gemischen unterschiedlicher Ausgangsmaterialien a) und/oder b) dem jeweils gewünschten Einsatzzweck angepaßt werden. Auf silylierten Phenolen und Bisphenolen basierende Diisocyanate haben zum Teil LC-Eigenschaften.

In den nachfolgenden Ausführungsbeispielen beziehen sich alle Prozentangaben auf Gewichtsprozente.

Beispiel 1

3-Isocyanatopropionsäurephenylester

In einem 100 ml Dreihalskolben mit Innenthermometer, Magnetrührstab, Tropftrichter und Rückflußkühler werden zu 0,1 Mol Isocyanatopropionsäurechlorid 0,1 Mol Phenoxytrimethylsilan zugetropft und eine Stunde bei 140 °C gerührt. Anschließend wird das entstandene Trimethylchlorsilan abgezogen und das Rohprodukt destillativ gereinigt.

Ausbeute an 3-Isocyantopropionsäurephenylester: 87 %

Kp.: 100 °C bei 0,04 mbar

IR-Spektrum: V(NCO) = 2.280 $cm^{-1}$
V(C=O) = 1.750 $cm^{-1}$

Beispiel 2

12-Isocyanatododecansäuremethylester

Man setzt, wie unter Beispiel 1 beschrieben, 0,1 Mol 12-Isocyanatodecansäurechlorid mit 0,11 Mol Methoxytrimethylsilan bei 75 °C um, bis die Säurechloridbande bei 1.800 $cm^{-1}$ verschwunden ist und erhält nach Destillation das gewünschte Produkt.

Ausbeute an 12-Isocyanatodecansäuremethylester: 67 %

Kp.: 100 °C bei 1,3 x $10^{-6}$ mbar (Kurzwegdestillation)

IR-Spektrum: V(NCO) = 2.270 $cm^{-1}$
V(C=O) = 1.740 $cm^{-1}$

Beispiele 3 bis 5

Reaktion mit silylierten Diolen

In einem 100 ml Stickstoffkolben werden 0,05 Mol silylierter Alkohol mit je 0,06 Mol Isocyanatocarbonsäurechlorid pro funktioneller Gruppe des jeweiligen Alkohols versetzt, ca. 30 Stunden auf 80 bis 100 °C erwärmt, bis die Säurechloridbande praktisch verschwunden ist. Anschließend wird das Produkt mit Hilfe einer Kurzwegdestillation im Hochvakuum gereinigt.

Beispiel 3

1,4-Bis-3-isocyanatopropionsäuretetramethylenester

Ausgangsmaterialien: 3-Isocyanatopropionsäurechlorid und O,O′-Bis-trimethylsilyl-tetramethylendiol
Ausbeute: 92 %
Kp.: 165 °C bei 9,3 x $10^{-6}$ mbar
IR-Spektrum: V(NCO) = 2.280 $cm^{-1}$
V(C=O) = 1.730 $cm^{-1}$

Beispiel 4

1,4-Bis-4-isocyanatobuttersäuretetramethylenester

Ausgangsmaterialien: 4-Isocyanatobuttersäurechlorid und das silylierte Diol gemäß Beispiel 3
Ausbeute: 89 %
Kp.: 170 °C bei 5,3 x $10^{-6}$ mbar
IR-Spektrum: V(NCO) = 2.280 $cm^{-1}$
V(C=O) = 1.730 $cm^{-1}$

Beispiel 5

1,4-Bis-6-isocyanatocapronsäuretetramethylenester

Ausgangsmaterialien: 6-Isocyanatocapronsäurechlorid und das silylierte Diol gemäß Beispiel 3
Ausbeute: 87 %
Kp.: 190 °C bei 9,3 x $10^{-6}$ mbar
IR-Spektrum: V(NCO) = 2.280 $cm^{-1}$
V(C=O) = 1.730 $cm^{-1}$

Beispiele 6 bis 8

Man verfährt, wie unter Beispiel 3 beschrieben, und erhält:

Beispiel 6

Trimethylolpropan-tris-(3-isocyanato-propionsäureester)

Ausgangsmaterialien: 3-Isocyanatopropionsäurechlorid und O,O′,O″-Tris-trimethylsilyl-trimethylolpropan
Ausbeute: 90 %
IR-Spektrum: V(NCO) = 2.280 $cm^{-1}$
V(C=O) = 1.735 $cm^{-1}$

Beispiel 7

Trimethylolpropantris-(4-isocyanatobuttersäureester)

Ausgangsmaterialien: 4-Isocyanatobuttersäurechlorid und das silylierte Triol gemäß Beispiel 6
Ausbeute: 95 %
IR-Spektrum: V(NCO) = 2.280 $cm^{-1}$
V(C=O) = 1.735 $cm^{-1}$

Beispiel 8

Trimethylolpropantris-(6-isocyanatocapronsäureester)

Ausgangsmaterialien: 6-Isocyanatocapronsäurechlorid und das silylierte Triol gemäß Beispiel 6
Ausbeute: 90 %

IR-Spektrum: V(NCO) = 2.280 $cm^{-1}$
V(C=O) = 1.735 $cm^{-1}$

Beispiele 9 bis 11

Reaktion mit silylierten vierwertigen Alkoholen

Man verfährt, wie unter Beispiel 3 beschrieben, und erhält:

Beispiel 9

Pentaerythrit-tetrakis-(3-isocyanatopropionsäureester)

Ausgangsmaterialien: 3-Isocyanatopropionsäurechlorid und O,O′,O″,O‴-Tetrakis-trimethylsilyl-pentaerythrit
Ausbeute: 90 %
IR-Spektrum: V(NCO) = 2.270 $cm^{-1}$
V(C=O) = 1.739 $cm^{-1}$

Beispiel 10

Pentaerythrit-tetrakis-(4-isocyanatobuttersäureester)

Ausgangsmaterialien: 4-Isocyanatobuttersäurechlorid und das silylierte Tetrol gemäß Beispiel 9
Ausbeute: 85 %
IR-Spektrum: V(NCO) = 2.280 $cm^{-1}$
V(C=O) = 1.740 $cm^{-1}$

Beispiel 11

Pentaerythrit-tetrakis-(6-isocyanatocapronsäureester)

Ausgangsmaterialien: 6-Isocyanatocapronsäurechlorid und das silylierte Tetrol gemäß Beispiel 9
Ausbeute: 80 %
IR-Spektrum: V(NCO) = 2.280 $cm^{-1}$
V(C=O) = 1.740 $cm^{-1}$

Beispiele 12 bis 14

Umsetzung mit silylierten Aminen und Aminoalkoholen

Beispiel 12

6-Isocyanatocapronsäure-N-butylamid

In einem 250 ml Reaktionskolben mit Innenthermometer, Magnetrührstab und Tropftrichter werden 0,1 Mol Isocyanatocapronsäurechlorid in 150 ml wasserfreiem Toluol vorgelegt. Das Gemisch wird auf -5 °C abgekühlt und tropfenweise mit 0,1 Mol n-Butylamino-trimethylsilan versetzt. Die Temperatur wird dabei unterhalb 5 °C gehalten. Nach beendeter Reaktion wird noch 30 Minuten bei Raumtemperatur gerührt, Toluol destillativ entfernt und anschließend das Produkt mit Hilfe einer Kugelrohrdestillationsanlage im Hochvakuum gereinigt.
Ausbeute an 6-Isocyanatocapronsäure-N-butylamid: 78 %
IR-Spektrum: V(NCO) = 2.280 $cm^{-1}$
V(NH) = 3.300 $cm^{-1}$ und 1.650 $cm^{-1}$

Beispiel 13

6-Isocyanatocapronsäure-N(2-trimethylsiloxy-ethyl)-amid aus O.N-Bistrimethylsilyl-2-aminoethanol und

6-Isocyanatocapronsäurechlorid,wie unter Beispiel 12 angegeben.
Ausbeute: 76 %
Kp.: 155 ° C bei 5,3 x $10^{-6}$ mbar
IR-Spektrum: V(NCO) = 2.280 $cm^{-1}$
V(NH) = 3.300 $cm^{-1}$ und 1.650 $cm^{-1}$

Beispiel 14

6-Isocyanatocapronsäure-N(4-trimethylsiloxy-butyl)-amid aus O.N-Bistrimethylsilyl-4-aminobutanol und 6-Isoanatocapronsäurechlorid, wie unter Beispiel 12 beschrieben:
Ausbeute: 81 %
Kp.: 165 ° C bei 5,3 x $10^{-6}$ mbar
IR-Spektrum: V(NCO) = 2.280 $cm^{-1}$
V(NH) = 3.300 $cm^{-1}$ und 1.650 $cm^{-1}$

Beispiele 15 bis 17

Darstellung linearer Polyurethane durch Umsetzung von Diesterdiisocyanaten mit 1,4 Butandiol

In einem 50 ml Rundkolben mit Magnetrührstab und Rückflußkühler werden 0,02 Mol 1,4-Butandiol und 15 ml wasserfreies Chlorbenzol und 0,02 Mol eines Diesterdiisocyanates zusammengegeben. Das trübe Flüssigkeitsgemisch wird bei Erwärmen auf 95 ° C klar. Dann wird noch eine Stunde bei 140 ° C gerückflußt und das Polyurethan heiß in Methanol ausgefällt, abgesaugt und bei 60 ° C im Ölpumpenvakuum getrocknet.

Beispiel 15

Umsetzung von 1,4-Bis-3-isocyanatopropionsäuretetramethylenester nach Beispiel 3 erhalten:
Staudinger-Index (bestimmt in Aceton bei 25 ° C):
[$\eta$] = 55,0 (ml/g)
IR-Spektrum: V(NCO) = keine Bande
V(C=O) = 1.730 $cm^{-1}$
V(NH) = 3.340 $cm^{-1}$ und 1.540 $cm^{-1}$
DSC-Messung: endothermer Peak bei 93 ° C

Beispiel 16

Umsetzung von 1,4-Bis-4-isocyanatobuttersäuretetramethylenester nach Beispiel 4 erhalten:
Staudinger-Index (bestimmt in Aceton bei 25 ° C):
[$\eta$] = 59,7 (ml/g)
IR-Spektrum: V(NCO) = keine Bande
V(C=0) = 1.725 $cm^{-1}$
V(NH) = 3.320 $cm^{-1}$ und 1.540 $cm^{-1}$
DSC-Messung: endothermer Peak bei 93 ° C

Beispiel 17

Umsetzung von 1,4-Bis-6-isocyanatocapronsäuretetramethylenester nach Beispiel 5 erhalten:
Staudinger-Index (bestimmt in Aceton bei 25 ° C):
[$\eta$] = 106,6 (ml/g)
IR-Spektrum: V(NCO) = keine Bande
V(C=0) = 1.730 $cm^{-1}$
V(NH) = 3.320 $cm^{-1}$ und 1.540 $cm^{-1}$
DSC-Messung: endothermer Peak bei 103 ° C

Beispiel 18

4,4'-Bis-(isocyanatoethylcarboxy)biphenyl

Ausgangsmaterialien: 4,4'-Bis-(trimethylsilyloxy)-biphenyl und 3-Isocyanatopropionsäurechlorid.
Herstellung: in Analogie zu Beispiel 1.
Ausbeute: 65 %.
Das Diisocyanat weist innerhalb des Temperaturbereichs von 135 bis 160 °C LC-Eigenschaften auf.

## Patentansprüche

**1.** Verfahren zur Herstellung von Ester- und/oder Amidgruppen aufweisenden Isocyanaten, dadurch gekennzeichnet, daß man

a) Isocyanatoalkylcarbonsäurechloride
mit
b) organischen Verbindungen, die mindestens eine silylierte alkoholische und/oder phenolische Hydroxylgruppe und/oder mindestens eine silylierte Aminogruppen aufweisen und ansonsten unter den jeweiligen Reaktionsbedingungen gegenüber Isocyanat- und Chlorcarbonylgruppen inert sind,

bei -20 °C bis +150 °C zur Reaktion bringt.

**2.** Verfahren gemäß Anspruch 1, dadurch gekennzeichnet, daß man als Komponente a) Verbindungen der allgemeinen Formel

OCN-R-COCl

verwendet, für welche

R für einen aliphatischen Kohlenwasserstoffrest mit 2 bis 5 Kohlenstoffatomen steht.

**3.** Verfahren gemäß Anspruch 1 und 2, dadurch gekennzeichnet, daß man als Komponente b) Verbindungen der allgemeinen Formel

$[R'_3Si\text{-}X]_m R''$

verwendet, für welche

R' für einen Alkylrest mit 1 bis 4 Kohlenstoffatomen steht,
R" für einen jeweils m-wertigen aliphatischen Kohlenwasserstoffrest mit 1 bis 18 Kohlenstoffatomen, cycloaliphatischen Kohlenwasserstoffrest mit 4 bis 15 Kohlenstoffatomen oder aromatischen Wasserstoffrest mit 6 bis 13 Kohlenstoffatomen steht,
X für Sauerstoff oder eine -NH-Gruppe steht
und
m eine ganze Zahl von 2 bis 4 bedeutet.

## Claims

**1.** A process for the production of isocyanates containing ester and/or amide groups, characterized in that
a) isocyanatoalkyl carboxylic acid chlorides
are reacted with
b) organic compounds which contain at least one silylated alcoholic and/or phenolic hydroxyl group and/or at least one silylated amino group and which are otherwise inert to isocyanate and chlorocarbonyl groups under the particular reaction conditions
at temperatures of -20 to +150 °C.

**2.** A process as claimed in claim 1, characterized in that compounds corresponding to the following general formula

OCN - R - COCl

in which

R represents an aliphatic hydrocarbon radical containing 2 to 5 carbon atoms,

are used as component a).

**3.** A process as claimed in claims 1 and 2, characterized in that compounds corresponding to the following general formula

$[R'_3Si\text{-}X]_m R''$

in which
R' is an alkyl radical containing 1 to 4 carbon atoms,
R" is an m-functional aliphatic aliphatic hydrocarbon radical containing 1 to 18 carbon atoms, cycloaliphatic hydrocarbon radical containing 4 to 15 carbon atoms or aromatic hydrocarbon radical containing 6 to 13 carbon atoms,
X is oxygen or an -NH-group
and
m is an integer of 2 to 4,
are used as component b).

## Revendications

**1.** Procédé de préparation d'isocyanates présentant des radicaux ester et/ou amide caractérisé en ce que

a) des chlorures d'acide isocyanatoalkylcarboxylique
sont amenés à réagir avec
b) des composés organiques qui présentent au moins un radical hydroxyle alcoolique et/ou phénolique silylé et/ou au moins un radical amino silylé et sont par ailleurs inertes par rapport aux radicaux isocyanate et chlorocarbonyle dans les conditions de réaction respectives,
à une température de l'ordre de -20 °C à +150 °C.

**2.** Procédé selon la revendication 1 caractérisé en ce que sont utilisés comme réactif a) des composés de formule générale

OCN-R-COCl

dans laquelle

R désigne un radical hydrocarbure aliphatique avec 2 à 5 atomes de carbone

**3.** Procédé selon les revendications 1 et 2 caractérisé en ce que sont utilisés comme réactif b) des composés de formule générale

$[R'_3Si\text{-}X]_m R''$

dans laquelle

R' désigne un radical alkyle avec 1 à 4 atomes de carbone,
R" désigne un radical hydrocarbure aliphatique de valence m avec 1 à 18 atomes de carbone, un radical hydrocarbure cycloaliphatique avec 4 à 15 atomes de carbone ou un radical hydrogène aromatique avec 6 à 13 atomes de carbone,
X désigne l'oxygène ou un radical -NH-
et
m désigne un nombre entier de 2 à 4.